# EUROPEAN PATENT SPECIFICATION

(11) **EP 4 193 935 B1**
(45) Date of publication and mention of the grant of the patent: **08.07.2026**
(21) Application number: 22210239.4
(22) Date of filing: 29.11.2022
(51) Int. Cl.: A61B 17/06

(54) **LIFTING THREAD IN IMPROVED MODEL**
HEBEFADEN IN EINEM VERBESSERTEN MODELL
FIL DE LEVAGE DANS UN MODÈLE AMÉLIORÉ

(30) Priority: 09.12.2021 KR 20210175741; 23.03.2022 KR 20220035957
(43) Date of publication of application: 14.06.2023
(73) Proprietor: Chang, Dooyeoul, Seoul 06200 (KR)
(72) Inventor: Chang, Dooyeoul, Seoul 06200 (KR)
(74) Representative: BCKIP Part mbB

(56) References cited:
- KR-B1- 101 926 311
- US-A- 5 931 855
- US-A1- 2009 112 259
- US-A1- 2013 079 815

## Description

This application claims priority to Korean Patent Application No. 10-2021-0175741 filed on December 09, 2021, No. 10-2022-0035957 filed on March 24, 2022 before the Korean Intellectual Property Office.

### Field of the Invention

The present invention relates to a lifting thread in an improved model, and more particularly, to a lifting thread in an improved model which is used in a surgical operation on the skin.

### Description of the Related Arts

A surgical operation for lifting is one of various surgical operation methods carried out for removing wrinkles, and improving skin sagging, and with an increase in interest in "antiaging" currently, as people's interest in skin care has been increasing largely, interest in a surgical operation for lifting which shows its effect immediately has also been increasing.

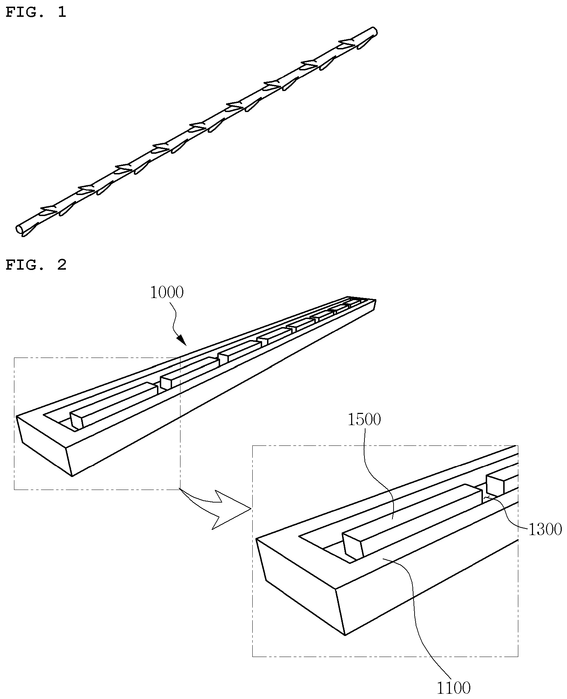

FIG. 1 illustrates a lifting thread according to a conventional art.

As illustrated, with respect to a surgical operation for lifting using the conventional lifting thread in which projections are formed on its surface, after the lifting thread is inserted into the skin using a tool, like a cannula, without cutting out the skin, when the inserted lifting thread is pulled, skin tissue is pulled by the projections formed in the lifting thread with pulling of the lifting thread, and when the lifting thread pulled as described above is fixed, a state of the skin tissue being pulled is maintained, so the skin tissue which sags or is wrinkled is restored from sagging, or wrinkles thereof are improved.

Such a surgical operation method for lifting using the thread is advantageous in that face regions can be corrected delicately, and since a cannula is used, the method enables a user to make a normal living rapidly relatively compared with a surgery method of directly cutting out the skin, and it is advantageous in that many problems caused by surgery, like the occurrence of a scar, can be solved.

However, with respect to the existing lifting thread, as illustrated in FIG. 1, since the projections having a thorn-like shape are entirely formed around an outer circumferential surface of the lifting thread whose diameter is small, when tensile strength is excessively applied to the lifting thread, it is problematic in that some subcutaneous tissue is damaged, and since the projections are entirely formed on the lifting thread, a phenomenon which shows that the tissue of a unintended region sinks in may occur, and a good effect is maintained in the beginning of the surgical operation for lifting, whereas with the lapse of time, the projections are pushed out from the skin tissue little by little, or a shape of the projections is transformed, so it is problematic in that the effect of lifting decreases gradually.

Also, since the existing lifting thread shows that the projections which strain at a ligament or a fibrous component melt rapidly, it has been problematic in that the effect of lifting decreases, and it has been pointed out that a side effect of the skin sinking in, occurs because the projections are inelastic.

KR 101926311 B1 discloses a suture comprising a plurality of cogs thereon, with the plurality of cogs being disposed on opposite sides of the suture and having different shapes and/ or orientations on different portions of the suture in order to conform to different loads applied by the soft tissue at the respective portions.

US 2013/079815 A1 discloses a surgical suture comprising projections on two opposite sides of the suture having a T-shape, with a wide column of the T-shape extending outwardly from the suture and small protrusions on either side of the column at an outer edge of the T-shape forming the head portion of the T-shape.

US 2009/112259 A1 and US 5931855 A both disclose a suture containing barbs which are disposed in a spiral configuration around the suture.

### SUMMARY OF THE INVENTION

The present invention has been devised for solving the aforesaid problems, and provides a lifting thread in an improved model which is capable of lengthening a lift space of the lifting thread by improving the shapes of projections and increasing a cross sectional area, and which are capable of improving a phenomenon of the skin sinking in by making the projections elastic.

Also, the lifting thread in the improved model according to the present invention comprises a lifting part and a fixed part, wherein the lifting part comprises thorn projections, and functions to lift the skin through the thorn projections, and the fixed part comprises binding projections, and functions to be fixed to the skin through the binding projections, and accordingly, the present invention is intended for solving the problem of the conventional art which shows that the effect of an existing lifting thread fails to be maintained for a long time, and is shortened.

Also, the lifting thread in an improved model according to the present disclosure is configured in variously specialized structures in which a length of the lifting part and a length of the fixed part become different according to each region of a user's face, and the user's face can entirely be lifted using only the lifting thread in the improved model according to the present invention.

More specifically, in case that the lifting thread in the improved model according to the present disclosure is used in the frontal, it functions to uplift a frontal skin located at an upper portion of eyebrows, in case that the lifting thread is used in the zygoma, it functions to uplift nasolabial creases (the Chinese character 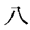 -like creases), in case that the lifting thread is used in the mandible, it is effective to improve marionette lines and is also intended for more improving an effect of lifting than that occurring from the existing lifting thread with respect to lifting on body parts, like the breast or the hip, as well as lifting on chin parts in addition to the improvement of the creases.

A lifting thread in an improved model according to one exemplary embodiment of the present disclosure intended for solving the aforesaid problems may comprise: a wire main-body 100 formed in a T-like shape; and binding projections 200 having a T-like shape integrally formed on an outer circumferential surface of the wire main-body 100.

According to another exemplary embodiment of the present disclosure, the lifting thread in the improved model may comprise a main body 10 which comprises: two outer frames 11 facing each other; a plurality of connection frames 13 configured to connect said two outer frames 11 to each other, and formed to be spaced apart from each other at fixed intervals; and slots 15 which are vacant spaces formed between each of the plurality of connection frames, and the binding projections 200 may be formed in such a manner as to cut the outer frames 11.

According to another exemplary embodiment of the present disclosure, the lifting thread in the improved model may further comprise thorn projections 150 integrally formed on another outer circumferential surface of the wire main-body 101, and configured to protrude to incline from a surface of the wire main-body 101 into a direction of one side.

According to another exemplary embodiment of the present disclosure, the lifting thread in the improved model may comprise: a lifting part A in which the thorn projections 150 are formed on one side of the wire main-body 101; and a fixed part B in which the binding projections 200 are formed on another side which is opposite to one side of the wire main-body 101.

According to another exemplary embodiment of the present disclosure, the plurality of thorn projections 150 may show that the thorn projections 150 are formed to be spaced apart from each other at fixed intervals in a spiral direction on a surface of the wire main-body 101.

According to another exemplary embodiment of the present disclosure, the plurality of binding projections 200 may show that the binding projections are formed to be spaced apart from each other at fixed intervals in a spiral direction on another surface of the wire main-body.

According to another exemplary embodiment of the present disclosure, the thorn projections 150 may be elastic, and may be formed to pull a ligament extending from a lower portion of the skin, or a fibrous component for lifting.

According to another exemplary of the present invention, the binding projections 200 may be elastic, and may be fixed to the skin.

According to another exemplary embodiment of the present disclosure, on the surface of the wire main-body 101, the thorn projections 150 are formed in such a manner as to cut the outer frames at a fixed angle with the surface of the wire main-body 101 as a standard.

A method of manufacturing a lifting thread in an improved model according to the other exemplary embodiment of the present disclosure may comprise: a step of preparing a mold 1000, a mold preparation step; a step of injecting a raw material (not drawn) into the mold 1000, a material injection step; a step of separating a main body completed after the lapse of fixed time from the mold 1000, a mold separation step; and a step of forming binding projections 200 by cutting out outer frames 11, a processing step.

According to the present disclosure, shapes of the projections are improved so that the projections can catch in a fibrous support under the skin more well, and a duration period for which the effect of a surgical operation is maintained can increase, and the projections are formed to be elastic so that a phenomenon which shows that the skin sinks in can be improved.

The lifting thread in the improved model according to the present disclosure can comprise a lifting part and a fixed part, wherein the lifting part comprises thorn projections, thereby functioning to lift the skin through the thorn projections, and the fixed part comprises binding projections, thereby functioning to be fixed to the skin through the binding projections.

Thus, the lifting thread in the improved model according to the present disclosure can solve the problem of a conventional art which shows that a duration period for an existing lifting thread fails to be maintained for a long time, and is shortened. Also, as a special surgical operation technique, like tagging or tying which has been used for extending the duration period for the lifting thread is substituted by the lifting thread in the improved model according to the present disclosure, an operator's convenience can be provided thanks to a more simple and easy method.

Also, since the lifting thread in the improved model according to the present disclosure can be configured in variously specialized structures in which a length of the lifting part and a length of the fixed part become different, a side effect, like the movement of an existing lifting thread occurring due to an insufficient length of a fixed part of the existing lifting thread, can be prevented from occurring, and since an effect of lifting from target regions having various lengths, which cannot be expected from the existing lifting thread, is maximized, a user's face can entirely be lifted using only the lifting thread in the improved model according the present disclosure.

More specifically, in case that the lifting thread in the improved model according the present disclosure is used in the frontal, it can function to uplift a frontal skin located at an upper portion of eyebrows, in case that the lifting thread is used in the zygoma, it can function to uplift nasolabial creases (the Chinese character 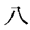 -like creases), in case that the lifting thread is used in the mandible, it can be effective to improve marionette lines, and in addition to this improvement, an effect of lifting on body parts, like the breast or the hip, as well as lifting on chin parts can be improved beyond an effect of lifting of the existing thread.

### BRIEF DESCRIPTION OF THE DRAWINGS

FIG. 1 illustrates a lifting thread according to a conventional art.
FIG. 2 is a view illustrating a lifting thread in an improved model according to one exemplary embodiment of the present disclosure.
FIG. 3 is a view for explaining a lifting part of the lifting thread in the improved model, and a manufacturing method of the lifting thread in the improved model according to another exemplary embodiment of the present disclosure.
FIG. 4 is a view for explaining a fixed part of the lifting thread in the improved model, and a manufacturing method of the lifting thread in the improved model according to another exemplary embodiment of the present disclosure.
FIG. 5 is a view illustrating a main body of the lifting thread in the improved model manufactured using a mold according to another exemplary embodiment of the present disclosure.
FIG. 6 is a view illustrating a lifting thread in an improved model according to another exemplary embodiment of the present disclosure.
FIG. 7 is a view illustrating a binding projection 200 of the lifting thread in the improved model according to another exemplary embodiment of the present invention.
FIG. 8 is a view illustrating the mold for manufacturing the lifting thread in the improved model according to another exemplary embodiment of the present disclosure.
FIG. 9 and FIG. 10 are views illustrating lifting threads in an improved model according to another exemplary embodiments of the present disclosure.
FIG. 11 and FIG. 12 are views illustrating each binding projection from the lifting threads in the improved model according to said another exemplary embodiments of the present invention.
FIG. 13 is a view illustrating a lifting thread in an improved model according to another exemplary embodiment of the present invention.
FIG. 14 is a view for explaining a lifting part of the lifting thread in the improved model, and a manufacturing method thereof according to another exemplary embodiment of the present invention.
FIG. 15 is a view for explaining how to use each lifting thread in an improved model according to the other exemplary embodiment of the present invention.

### DETAILED DESCRIPTION OF THE INVENTION

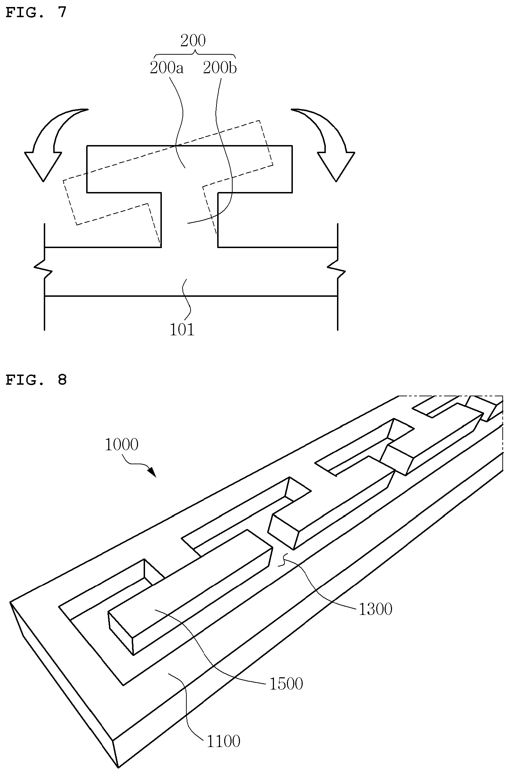

The invention is set forth in the embodiments of figures 7, and 11 to 15, with the other embodiments being useful for understanding the invention.

Various modifications for the present invention may be made, the present invention may have several exemplary embodiments, and specific exemplary embodiments will be exemplified in the drawings and will be described in detail in the detailed description. However, the exemplary embodiments should not be construed as limiting the present invention to specifically embodied forms, but should be understood as including all the modifications, equivalents, or alternations included in the technical idea and scope of the present invention, as set out in the appended claims.

However, in the following description with regard to embodied forms, when it is judged that the detailed description of conventional functions or constitutions related with the present invention may make the gist of the present invention unnecessarily unclear, the detailed description thereof will be omitted. Also, the respective sizes of the constituent elements shown in the drawings may exaggeratedly be drawn for the description, and do not mean actually applied sizes.

Also, throughout the specification, when it is described that one constituent element "is connected" to or "is linked with" another constituent element, although it may be understood that said one constituent element is directly connected to or is directly linked with said another constituent element, it should be understood that said one constituent element may be connected to or may be linked with said another constituent element via the other constituent element therebetween, unless the context describing the present invention clearly indicates otherwise. Also, throughout the specification, when it is described that a part "comprises" a constituent element, this means that the part may further comprise other constituent elements rather than excluding the other constituent elements, unless the context clearly indicates otherwise.

Various modifications for the present invention may be made, the present invention may have several exemplary embodiments, and specific exemplary embodiments will be exemplified in the drawings and will be described in detail in the detailed description. However, the exemplary embodiments should not be construed as limiting the present invention to specifically embodied forms, but should be understood as including all the modifications, equivalents, or alternations included in the technical idea and scope of the present invention, as set out in the appended claims.

The present disclosure provides a lifting thread in an improved model 100 comprising: a wire main-body 101 formed in a straight line-like shape; and binding projections 200 having a T-like shape integrally formed on an outer circumferential surface of the wire main-body 101.

Furthermore, the present invention provides the lifting thread 100 in the improved model which is characteristic in that the binding projections 200 are elastic, and pull an extending ligament located at a lower portion of the skin, or a fibrous component for lifting, and with respect to the lifting thread 100 in the improved model, the binding projections 200 are formed in such a manner as to cut outer frames 11 from the main body 10 which comprises: two outer frames 11 facing each other; a plurality of connection frames 13 configured to connect said two outer frames 11 to each other, and formed to be spaced apart from each other at fixed intervals; and slots 15 which are vacant spaces formed between each of the plurality of connection frames 13.

Furthermore, when the lifting thread in the improved model is manufactured, a method of manufacturing the lifting thread in the improved model, which comprises: a step of preparing a mold 1000, a mold preparation step; a step of injecting a raw material (not drawn) into the mold 1000, a material injection step; a step of separating the main body 10 completed after the lapse of fixed time from the mold 1000, a mold separation step; and a step of forming the binding projections 200 by cutting the outer frames 11, a processing step, is provided.

Hereinafter, the concepts of the present disclosure as described above are described in more detail with reference to exemplary embodiments, which are preferably embodied, as well as the accompanying drawings.

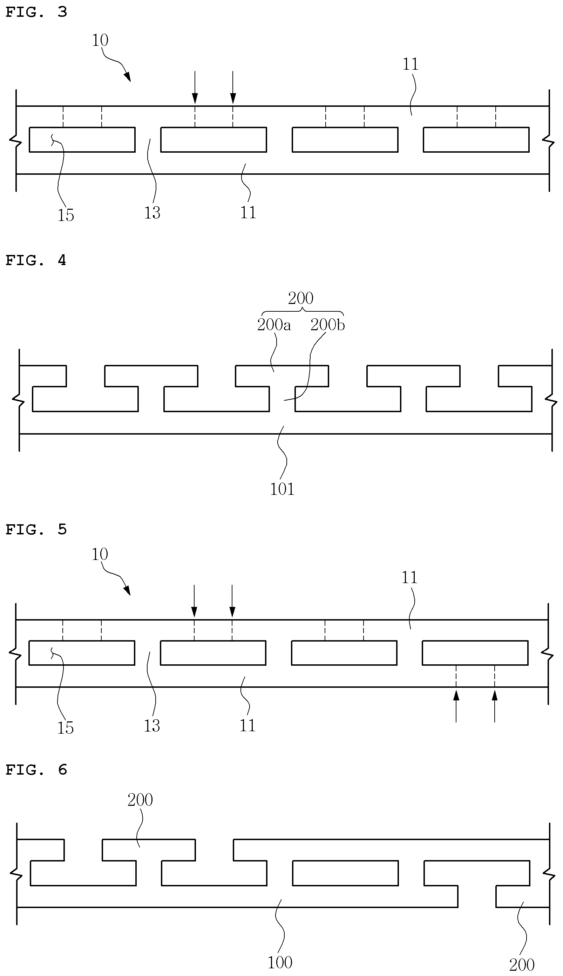

FIG. 2 is the view illustrating a mold for manufacturing the lifting thread in the improved model according to one exemplary embodiment of the present disclosure, FIG. 3 is the view illustrating a main body of the lifting thread in the improved model manufactured using the mold according to another exemplary embodiment of the present disclosure, and FIG. 4 is the view illustrating the lifting thread in the improved model according to another exemplary embodiment of the present disclosure.

As illustrated in FIG. 2, the mold 1000 comprises: an outer wall mold 1100 configured to surround an outer portion; a material injection part 1300 formed by being down in level; and a plurality of inner molds 1500 formed in the inside of the material injection part 1300, and formed in the same level as that of the outer wall mold 1100.

The mold 1000 may be manufactured of an elastic body so as to facilitate separation from the main body 10.

With respect to a raw material (not drawn) which constitutes the main body 10, it is preferable to use a material for medical treatment, like nylon, prolene, and so on, that is harmful to the human body, and secures flexibility and durability.

As illustrated in FIG. 3, the lifting thread in the improved model according to the present disclosure is characteristic in that the binding projections 200 are formed in such a manner as to cut the outer frames 11 as illustrated in FIG. 4 from the main body 10 comprising two outer frames facing each other, the plurality of connection frames 13 configured to connect said two outer frames 11 to each other, and formed to be spaced apart from each other at fixed intervals, and the slots 15 which are vacant spaces formed between each of the plurality of connection frames 13.

That is, as shown in FIG. 3, a wire main-body 101 of the lifting thread in the improved model 100 may be configured to comprise two outer frames 11 facing each other, the plurality of connection frames 13 configured to connect said two outer frames 11 to each other, and formed to be spaced apart from each other at fixed intervals, and slots 15 which are vacant spaces formed between each of the plurality of the connection frames 13.

Accordingly, as illustrated in FIG. 4, a part of the outer frame 11 located at one side is cut, so the binding projection 200 may be formed.

FIG. 5 is a view illustrating a main body of the lifting thread in the improved model manufactured using a mold according to another exemplary embodiment of the present disclosure, FIG. 6 is a view illustrating a lifting thread in an improved model according to another exemplary embodiment of the present disclosure, and FIG. 7 is a view illustrating the binding projection 200 of the lifting thread in the improved model according to another exemplary embodiment of the present invention.

According to another exemplary embodiment of the present disclosure, as illustrated in FIG. 5, the wire main-body 101 of the lifting thread in the improved model 100 may comprise: two outer frames facing each other; a plurality of connection frames 13 configured to connect said two outer frames to each other, and formed to be spaced apart from each other at fixed intervals; and slots 15 which are vacant spaces formed between each of the plurality of connection frames 13, and as illustrated in FIG. 6, the binding projections 200 may be formed in such a manner as to cut a part of each outer frame 11 located at both sides selectively.

In case of a conventional lifting thread, since projections which pull a ligament extending from a lower portion of the skin, or a fibrous component melted rapidly, it was problematic in that an effect of lifting became weaker, and since the projections were not elastic, a side effect of the skin sinking in occurred.

However, with respect to the lifting thread in the improved model according to another exemplary embodiment of the present invention shown in FIG. 7, as the binding projections 200 whose shape is improved in a T-like shape are formed, a cross sectional area increases so that the durability of melting can be lengthened.

Also, the binding projection 200 may comprise a head part 200A and a column part 200B, and the projection may be bent or rotated with elasticity in a state of the ligament or the fibrous component being caught thereon.

Accordingly, a side effect which shows that skin tissue gets torn, or some skin tissue is spoiled due to tension acting on the lifting thread 100 may be prevented from occurring, and the phenomenon of the skin and tissue sinking in may be prevented.

FIG. 8 is a view illustrating a mold for manufacturing the lifting thread in the improved model according to another exemplary embodiment of the present disclosure.

The lifting thread in the improved model according to another exemplary embodiment of the present disclosure may be manufactured using the mold 1000 illustrated in FIG. 8.

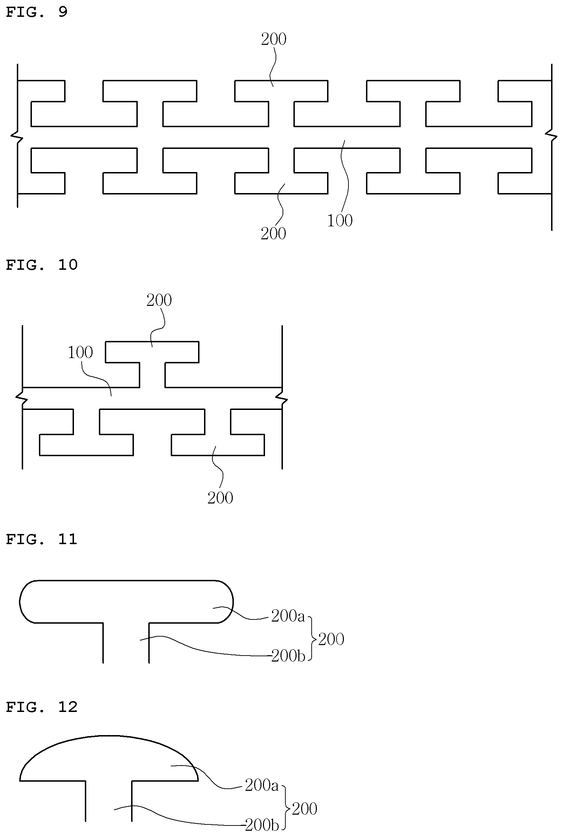

FIG. 9 and FIG. 10 are views illustrating lifting threads in an improved model according to another exemplary embodiments of the present disclosure.

According to the exemplary embodiment illustrated in FIG. 9 one wire main-body 100 is located in the center, and binding projections 200 are formed at an upper portion and a lower portion of the wire main-body. At this time, the binding projections 200 located at the upper portion and the binding projections 200 located at the lower portion may be formed to be identical to each other with respect to their central axis, or as illustrated in FIG. 10, the binding projections 200 located at the upper portion and the binding projections 200 located at the lower portion may be formed to cross with each other with respect to their central axis.

Also, according to the exemplary embodiments illustrated in FIG. 9 and FIG. 10, the method of forming the binding projections 200 by cutting the outer frames 11 may also be used, and the binding projections 200 may be formed using a mold directly.

FIG. 11 and FIG. 12 are views illustrating the binding projections with respect to the lifting threads in the improved model according to another exemplary embodiment of the present invention.

Since the binding projections 200 according to another exemplary embodiment of the present invention are elastic, and pull a ligament extending from a lower portion of the skin, or a fibrous component in a state of catching them, in order for the ligament or the fibrous component to be prevented from being damaged, as illustrated in FIG. 11, a head part 200A of the binding projection 200 may be configured in a round shape, and as illustrated in FIG. 12, the head part 200A of the binding projection 200 may be formed to make a curved line from both end portions to the outside.

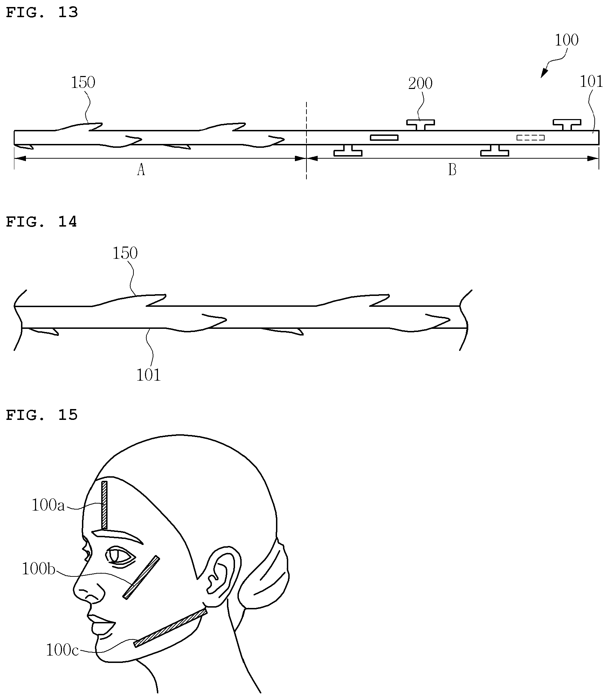

FIG. 13 is a view illustrating a lifting thread in an improved models according to another exemplary embodiment of the present invention, and FIG. 14 is a view for explaining a lifting part of the lifting thread in the improved model, and a manufacturing method of the lifting thread in the improved model according to another exemplary embodiment of the present invention.

Referring to FIG. 13, the lifting thread in the improved model 100 according to another exemplary embodiment of the present invention comprises: a wire main-body 101; thorn projections 150; and binding projections 200.

The wire main-body 101 may be formed in a wire form formed in a straight line-like shape, and the thorn projections 150 and the binding projections 200 may be formed on the wire main-body 101.

The thorn projections 150 are formed integrally on an outer circumferential surface of the wire main-body 101, thereby protruding to incline from a surface of the wire main-body 101 into a direction of one side, or protruding from the surface of the wire main-body 101 into a direction of rotation.

Also, the binding projections may be formed in a T-like shape and integrally on another outer circumferential surface of the wire main-body 101, or may be formed to protrude from a surface of the wire main-body 101 into a direction of rotation.

More specifically, the lifting thread in the improved model 100 may comprise: a lifting part A in which the thorn projections 150 are formed on one side of the wire main-body 101; and a fixed part B in which the binding projections 200 are formed on another side which is opposite to one side of the wire main-body 101.

At this time, a length of the lifting part A in which the thorn projections 150 are formed, and a length of the fixed part B in which the binding projections 200 are formed may be configured variously.

More particularly, when the length of the fixed part B in which the binding projections are formed is regularly maintained, and the length of the lifting part A in which the thorn projections 150 are formed is adjusted, many regions of the human body may be lifted.

Meanwhile, the plurality of thorn projections 150 may be configured in such a manner that the thorn projections 150 are formed to be spaced apart from each other at fixed intervals from the surface of the wire main-body 101 into a spiral direction, and in the same manner, the plurality of binding projections 200 may be configured in such a manner that the binding projections 200 are formed to be spaced apart from each other at fixed intervals from a surface of the wire main-body 101 into a spiral direction.

The binding projections 200 are elastic, and are able to be fixed to the skin, and the thorn projections 150 are elastic, and are able to function to pull a ligament extending from the lower portion of the skin, or a fibrous component for lifting.

Meanwhile, referring to FIG. 14, on a surface of the wire main-body 101, the thorn projections 150 may be formed to make a fixed angle with the surface of the wire main-body 101 as a standard, and this wire main-body 101 and the thorn projections 150 may be formed using molding.

FIG. 15 is a view for explaining how to use each lifting thread in an improved model according to the other exemplary embodiment of the present invention.

The lifting thread in the improved model 100 according to the other exemplary embodiment of the present invention comprises: a lifting part A; and a fixed part B.

The lifting part A may be configured to comprise thorn projections 150, thereby functioning to pull the skin through the thorn projections 150 for lifting, and the fixed part B may be configured to comprise binding projections 200, thereby functioning to be fixed to the skin through the binding projections 200.

Thus, the lifting thread in the improved model 100 according to the other exemplary embodiment of the present invention may solve a problem occurring from the conventional art which shows that a duration period for the existing lifting thread fails to be maintained for a long time, and is shortened.

Also, with respect to the lifting thread in the improved model 100 according to the other exemplary embodiment of the present invention, a length of the lifting part A and a length of the fixed part B become different according to each region of a user's face so that his or her face can entirely be lifted using only the lifting thread in the improved model 100 according to the other exemplary embodiment of the present invention.

For example, in case of a lifting thread in an improved model 100A is used in the frontal, it may function to uplift a frontal skin located at an upper portion of eyebrows, in case of a lifting thread in an improved model 100B is used in the zygoma, it may function to uplift nasolabial creases (the Chinese character 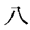 -like creases), and in case of a lifting thread in an improved model 100C used in the mandible, it may be effective to improve marionette lines.

That is, the length of the lifting part A in which the thorn projections 150 are formed, and the length of the fixed part B in which the binding projections 200 are formed may be configured variously, and when the length of the fixed part B in which the binding projections 200 are formed is regularly maintained, and the length of the lifting part A in which the thorn projections 150 are formed is adjusted, various regions of the human body may be lifted.

**[Table 1]**

| **Result Value of Fixing Strength (Unit: N)** | | | | | | | |
|---|---|---|---|---|---|---|---|
| **Subject Area** | **1** | **2** | **3** | **4** | **5** | **6** | **Average** |
| P | 58.2 | 45.2 | 35 | 32.6 | 26.8 | 57 | 42.5 |
| R | 8 | 19.2 | 26.8 | 11.8 | 10.6 | 25.6 | 17.0 |

Table 1 is a table for explaining a result of making an experiment in fixing strength of the lifting thread R according to the conventional art as illustrated in FIG. 1, and fixing strength of the lifting thread P in the improved model according to the other exemplary embodiment of the present invention as illustrated in FIG. 7. This experiment in the fixing strength was carried out in such a manner as to measure their fixing strength until the binding projections of each lifting thread cause a change in their form by being turned upside down (being peeled), being bent, or the like, rather than measuring physical properties of a medium which constitutes each lifting thread.

Referring to Table 1, it can be found that the fixing strength N of the lifting thread P in the improved model according to the other exemplary embodiment of the present invention is about 2.5 times higher than the fixing strength N of the lifting thread R according to the conventional art.

As previously described, in the detailed description of the invention have been described the detailed exemplary embodiments of the invention. However, various modifications can be made without deviating from the scope of the present invention. The technical idea of the present invention should not be construed as being limited to the aforesaid exemplary embodiments of the present invention, but should be decided on the basis of equivalents of the claims as well as the claims.

## Claims

1. A lifting thread (100) in an improved model, comprising:
a) a wire main-body (101) formed in a straight line-like shape and having an outer circumference surface;
b1) a lifting part (A) in which a plurality of thorn projections (150) are formed on one side of the wire main-body (101); and
b2) a fixed part (B) in which a plurality of binding projections (200) are formed on another side which is opposite to the one side of the wire main-body (101); wherein
c1) the plurality of thorn projections (150) show that the thorn projections (150) are formed to be spaced apart from each other at fixed intervals in a spiral direction on the outer circumference surface of the wire main-body (101) in the lifting part (A) ;
c2) the plurality of binding projections (200) have a T-like shape and are integrally formed spaced apart from each other at fixed intervals in a spiral direction on the outer circumferene surface of the wire main-body (101) in the fixed part (B); and
d) wherein each T-shape binding projection (200) is elastic and comprises a head part (200A) and a column part (200B) such that the T-shape binding projection (200) may be bent or rotated with elasticity in a state of a ligament or a fibrous component being caught thereon.

2. A lifting thread (100) according to claim 1, wherein the head part (200A) of the T-shape binding projection (200) is configured in a round shape.

3. A lifting thread (100) according to claim 1, wherein the head part (200A) of the T-shape binding projection (200) is formed to make a curved line from both end portions to the outside.

## Patentansprüche

1. Liftingfaden (100) in verbesserter Ausführung, umfassend:
a) einen Drahthauptkörper (101), der in geradliniger Form ausgebildet ist und eine äußere Umfangsoberfläche aufweist;
b1) einen Hebeteil (A), in dem eine Mehrzahl von Dornvorsprüngen (150) auf einer Seite des Drahthauptkörpers (101) ausgebildet ist; und
b2) einen Fixierteil (B), in dem eine Mehrzahl von Befestigungsvorsprüngen (200) auf der anderen Seite, die der einen Seite des Drahthauptkörpers (101) gegenüberliegt, ausgebildet ist; wobei
c1) die Mehrzahl von Dornvorsprüngen (150) so ausgebildet ist, dass die Dornvorsprünge (150) auf der äußeren Umfangsoberfläche des Drahthauptkörpers (101) im Hebeteil (A) in festen Abständen voneinander in spiralförmiger Richtung angeordnet sind;
c2) die Mehrzahl von Befestigungsvorsprüngen (200) eine T-Form aufweist und auf der äußeren Umfangsoberfläche des Drahthauptkörpers (101) im Fixierteil (B) in festen Abständen voneinander in spiralförmiger Richtung einstückig ausgebildet ist; und
d) wobei jeder T-förmige Befestigungsvorsprung (200) elastisch ist und ein Kopfteil (200A) und ein Stammteil (200B) umfasst, so dass der T-förmige Befestigungsvorsprung (200) in einem Zustand, in dem ein Band oder ein faseriger Bestandteil daran eingehängt ist, elastisch gebogen oder gedreht werden kann.

2. Liftingfaden (100) nach Anspruch 1, wobei das Kopfteil (200A) des T-förmigen Befestigungsvorsprungs (200) in runder Form ausgebildet ist.

3. Liftingfaden (100) nach Anspruch 1, wobei das Kopfteil (200A) des T-förmigen Befestigungsvorsprungs (200) so ausgebildet ist, dass es von beiden Endabschnitten nach außen eine gekrümmte Linie bildet.

## Revendications

1. Fil de levage (100) suivant un modèle amélioré, comprenant :
a) un corps de fil principal (101) de forme rectiligne et ayant une surface circonférentielle extérieure ;
b1) une partie de levage (A) où est formée une pluralité de saillies en épines (150) sur un premier côté du corps de fil principal (101) ; et
b2) une partie fixe (B) où est formée une pluralité de saillies de liaison (200) sur un autre côté opposé au premier côté du corps de fil principal (101) ; où
c1) la pluralité de saillies en épines (150) montre que lesdites saillies en épines (150) sont formées de manière espacées les unes des autres à intervalles fixes dans une direction hélicoïdale sur la surface circonférentielle extérieure du corps de fil principal (101) dans la partie de levage (A) ;
c2) la pluralité de saillies de liaison (200) ont une forme en T et sont formées d'un seul tenant, espacées les unes des autres à intervalles fixes dans une direction hélicoïdale sur la surface circonférentielle extérieure du corps de fil principal 101) dans la partie fixe (B) ; et
d) où chaque saillie de liaison en forme de T (200) est élastique et comprend une partie de tête (200A) et une partie de colonne (200B), permettant à ladite saillie de liaison en forme de T (200) d'être pliée ou tournée avec élasticité dans un état de fixation d'un ligament ou d'un composant fibreux sur celle-ci.

2. Fil de levage (100) selon la revendication 1, où la partie de tête (200A) de la saillie de liaison en forme de T (200) est prévue avec une forme arrondie.

3. Fil de levage (100) selon la revendication 1, où la partie de tête (200A) de la saillie de liaison en forme de T (200) est formée de manière à obtenir une ligne courbe depuis ses deux parties d'extrémité vers l'extérieur.
